(19) Europäisches Patentamt
European Patent Office
Office européen des brevets

(11) Publication number: **0 064 797 B1**

# (12) EUROPEAN PATENT SPECIFICATION

(45) Date of publication of patent specification: **22.05.85**

(51) Int. Cl.$^4$: **C 07 D 205/08**

(21) Application number: **82200761.3**

(22) Date of filing: **30.06.80**

(60) Publication number of the earlier application in accordance with Art. 76 EPC: **0 023 097**

## (54) N-Diarylmethyl-3-amino-4-alkenaryl-2-azetidinones and their preparation.

(43) Date of publication of application:
**17.11.82 Bulletin 82/46**

(45) Publication of the grant of the patent:
**22.05.85 Bulletin 85/21**

(84) Designated Contracting States:
**BE CH DE FR GB IT LI**

(56) References cited:
**US-A-4 166 816**

**TETRAHEDRON LETTERS, no. 30, July 1979, pages 2771-2774, Oxford, G.B. A.K. BOSE et al.: "Non-hazardous synthesis of isocephosporin intermediates via alpha-vinylamino-beta-lactams"**

(73) Proprietor: **SUMITOMO CHEMICAL COMPANY, LIMITED**
**15 Kitahama 5-chome Higashi-ku**
**Osaka-shi Osaka 541 (JP)**

(72) Inventor: **Sunagawa, Makoto**
**No. 11-8-106, Sonehigashi-machi 2-chome**
**Toyonaka-shi Osaka (JP)**
Inventor: **Matsumura, Haruki**
**No. 10-2-221, Sonehigashi-machi 2-chome**
**Toyonaka-shi Osaka (JP)**
Inventor: **Inoue, Takaaki**
**No. 14-7, Mefu 2-chome**
**Takarazuka-shi Hyogo (JP)**
Inventor: **Hirohashi, Toshiyuku**
**No. 5-F-1107, Misawa-cho**
**Ibaraki-shi Osaka (JP)**

(74) Representative: **Diamond, Bryan Clive**
**Gee & Co. Chancery House Chancery Lane**
**London WC2A 1QU (GB)**

**EP 0 064 797 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process of preparing certain β-lactams (azetidinones) which are useful as intermediates in the synthesis of pharmaceutically useful agents, and to the novel β-lactams.

In Tetrahedron Letters, No. 30 (1979), at page 2771, A. K. Bose *et al* describes the synthesis, from an (α-methyl-β-methoxycarbonyl)vinylaminoacetic acid:

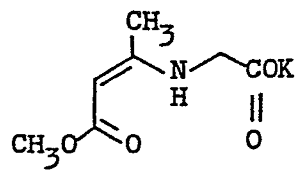

(1)

and an azomethine compound (Schiff base) derived from cinnamaldehyde and veratrylamine:

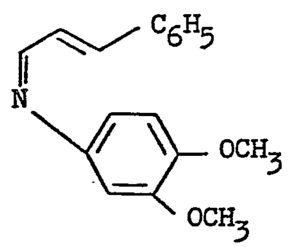

(2)

in the presence of ethyl chloroformate and triethylamine, of the product

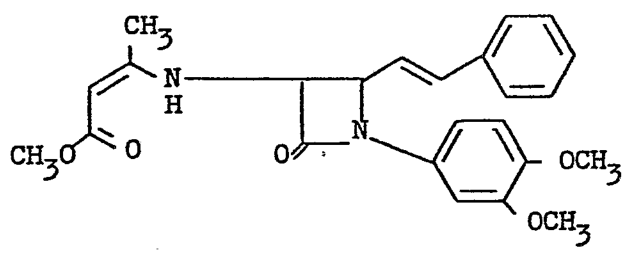

(3)

However, since the yield was low, and since the product (3) was an oil, it was necessary to recover it by silica gel chromatography. This synthesis of a product usable in the synthesis of isocephalosporins is therefore not readily used on an industrial scale.

We have now devised a new synthesis of this type whereby the azetidinone ring is synthesized, which provides novel intermediate compounds in good yield and with easy recovery.

The processes according to the invention are represented by the following reactions:

carboxylic acid: azomethine compound:

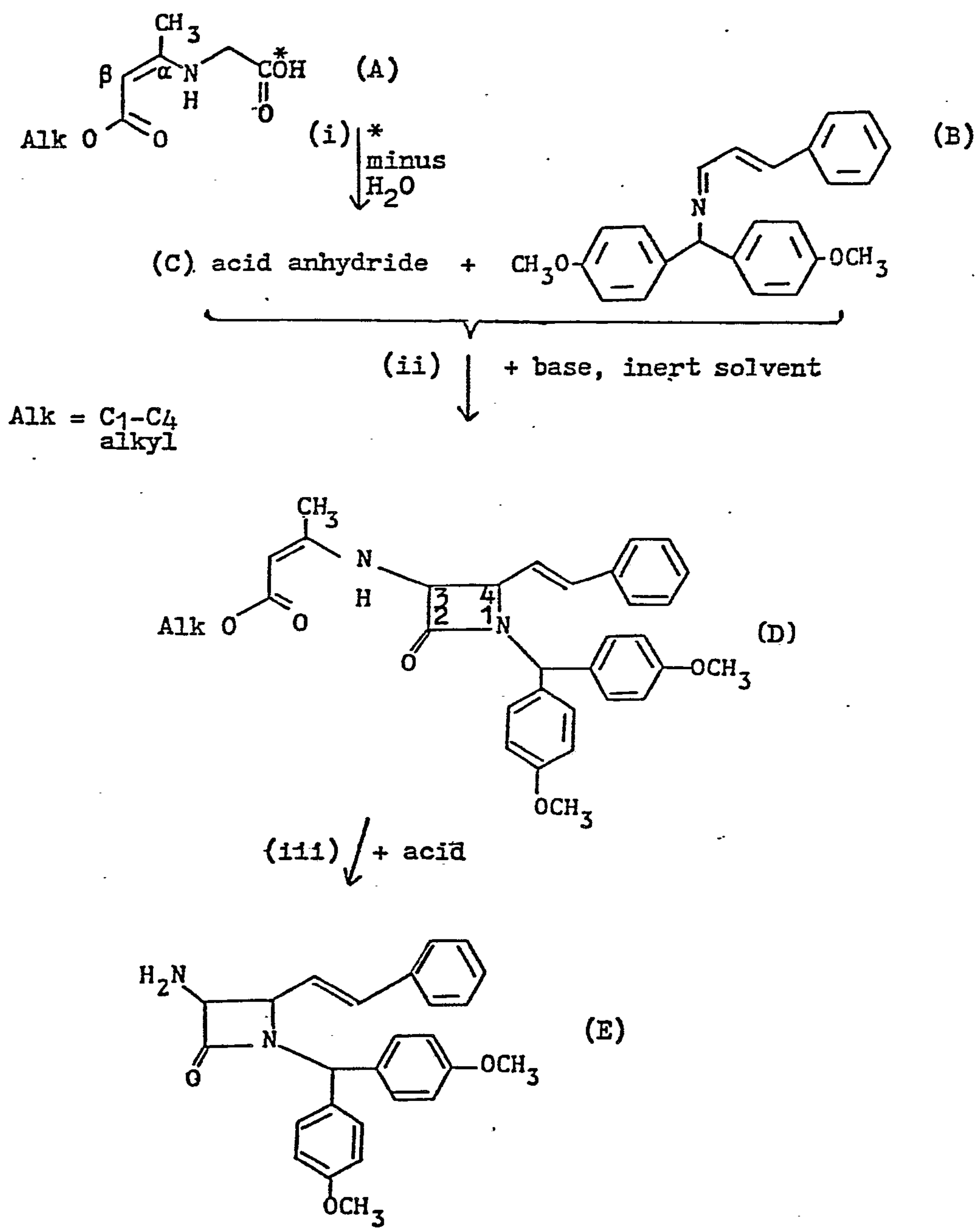


The azetidinones (D) and (E) are novel compounds. Acid addition salts can be formed of compound (D).

The process of the invention thus comprises preparing an N-diaryl-3-(substituted amino)-4-methylvinylbenzyl-2-azetidinone of the formula (D). An active acid anhydride derivative (C) is prepared from an (α-methyl-β-alkoxycarbonyl)vinylaminoacetic acid of the formula (A), wherein the alkyl group can, e.g., be methyl or ethyl; the derivative (C) can be prepared (i) by various known processes, e.g. by a mixed anhydride process which uses a chloroformate ester such as ethyl chloroformate. In the reaction (ii) with the azomethine compound (B), triethylamine is preferred as the base but other bases such as pyridine or lutidine may be used. The reaction is conducted in an inert solvent such as dichloromethane, optionally mixed with 1,2-dichloroethane, chloroform or toluene.

The further step (iii) of the process consists in treating the compound (D) with an acid, e.g. *p*-toluene sulfonic acid, to give the 4-unsubstituted amino derivative (E).

Compound (E) can by acylated to produce various amido derivatives, e.g. with an acylating agent such as trichloromethyl chloroformate or thienylacetyl chloride in the presence of a base, as described in our European Patent Publication No. 0 023 097.

The following Examples 1 and 2 illustrate steps (i) and (ii) of the process of the invention, and Example 3 illustrates step (iii).

Example 1

To N-(2-methoxycarbonyl-1-methylethenyl)glycine potassium salts (63.3 g) and triethylamine (30.3 g) in dry dichloromethane (1,250 ml) was added dropwise ethyl chloroformate (32.7 g) in dry dichloromethane (100 ml) at −20°C, and stirred for 30 minutes at the same temperature. To the reaction mixture was added dropwise *trans*-cinnamilidene-di(*p*-anisyl)methylamine (53.55 g) in dry dichloromethane (150 ml) at the same temperature, and the reaction mixture was stood overnight at room temperature and filtered over the filter aid Celite. The filtrate was washed with aqueous sodium bicarbonate (4%) and then water, dried and evaporated in vacuo to give crude crystals, which were recrystallized from diisopropyl ether to give pure crystals of 4-(2-phenylethenyl)-3-(2-methoxycarbonyl-1-methylethenyl)amino-N-di(*p*-anisyl)methyl-2-azetizinone (76%). m.p. 164—166°C.

$IR_{max}^{nujol}$ ($cm^{-1}$): 1737, 1650, 1606, 1457, 1370, 1263, 1155, 1025, 957, 827.

The starting material, *trans*-cinnamilidene-di-(*p*-anisyl)methylamine, was prepared from *trans*-cinnamaldehyde and di(*p*-anisyl)methylamine in a procedure similar to that described in Example 48 of EPA 0 023 097.

Example 2

4-(2-phenylethenyl)-3-(2-ethoxycarbonyl-1-methylethenyl)amino-N-di(*p*-anisyl)methyl-2-azetizinone was also prepared by using N-(2-ethoxycarbonyl-1-methylethenyl)glycine potassium salts instead of N-(2-methoxycarbonyl-1-methylethenyl)glycine potassium salts in a procedure similar to that described in Example 1 (87%). m.p. 122—124°C.

Example 3

To 4 - (2 - phenylethenyl) - 3 - (2 - methoxycarbonyl - 1 - methylethenyl)amino-N-di(*p*-anisyl)methyl-2-azetizinone (58.7 g) in dioxane (1,174 ml) were added *p*-toluenesulfonic acid monohydrate (23.96 g) and water (12.4 ml) and stood overnight at room temperature. The mixture was cooled with ice-water, and precipitates were collected by filtration and dried under reduced pressure to give, as white powder, 4-(2-phenylethenyl)-3-amino-N-di(*p*-anisyl)methyl-2-azetizinone, *p*-toluenesulfonic acid salts. m.p. 172—174°C.

$IR_{max}^{nujol}$ ($cm^{-1}$): 1760, 1610, 1508, 1460, 1373, 1245, 1175, 1030, 1003.

"Nujol" is a registered Trade Mark for a liquid paraffin used as solvent in the IR spectral determinations.

**Claims**

1. A β-lactam having the following general formula:.

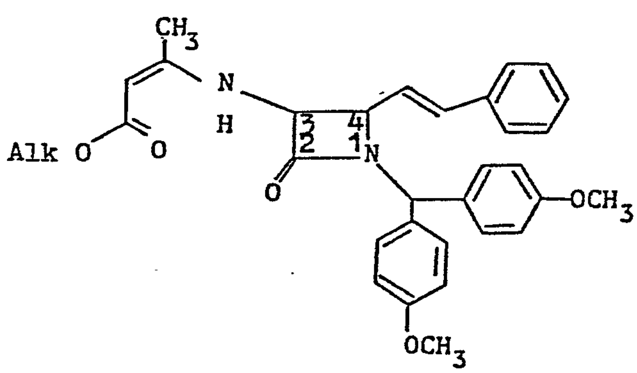

(D)

wherein

Alk represents an alkyl group having 1 to 4 carbon atoms.

2. A compound as claimed in Claim 1, wherein Alk is a methyl group or an ethyl group.

3. A process of preparing a compound as claimed in Claim 1 or 2, wherein an active acid anhydride derivative of a carboxylic acid compound having the following general formula:

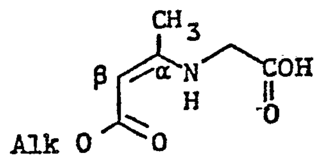

(A)

wherein
Alk is as defined in Claim 1 or 2, is reacted with an azomethine compound having the following formula:

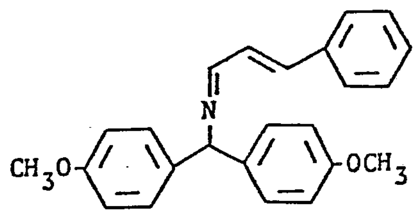

(B)

in the presence of a base.

4. A process as claimed in Claim 3, wherein the base is triethylamine, pyridine or lutidine.

5. A β-lactam having the following general formula:

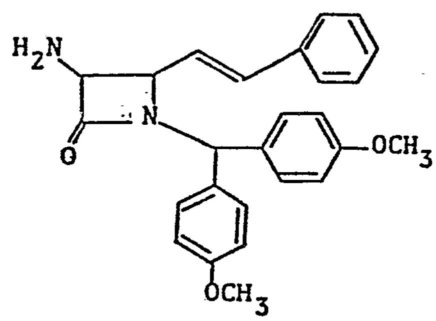

(E)

or an acid addition salt thereof.

6. A process of preparing a β-lactam as claimed in Claim 5, which comprises treating a compound as claimed in Claim 1 or 2 with an acid.

**Patentansprüche**

1. β-Laktam, das die folgende generelle Formel hat,

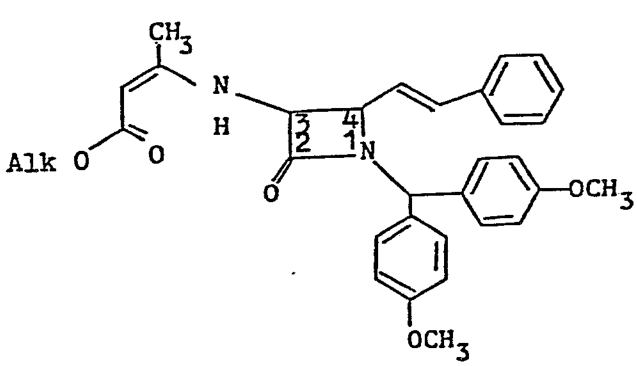

(D)

worin Alk eine Alkylgruppe mit 1 bis 4 Kohlenstoffatomen bedeutet.

2. Verbindung gemäss Anspruch 1, worin Alk eine Methylgruppe oder eine Aethylgruppe ist.

3. Verfahren für die Vorbereitung einer wie im Anspruch 1 oder 2 beanspruchten Verbindung, worin ein aktives Säureanhydrid Derivat von einer Carbonsäure Verbindung der folgenden generallen Formel,

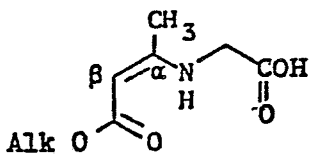

(A)

worin Alk wie im Anspruch 1 oder 2 definiert ist, mit einer Azomethin Verbindung der folgenden Formel

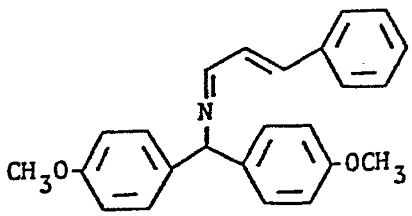

(B)

in Gegenwart einer Base reagiren gelassen wird.

4. Verfahren gemäss Anspruch 3, worin die Base Triethylamin, Pyridin oder Lutidin ist.

5. β-Laktam, das die folgende generelle Formel hat:

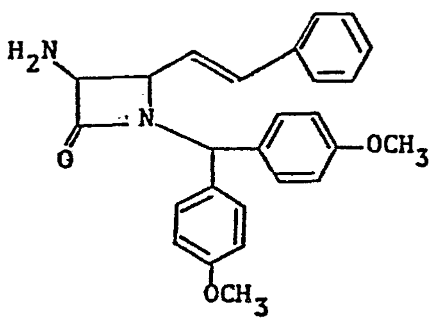

(E)

oder dessen Säurezusatzsalt.

6. Verfahren für die Vorbereitung eines wie im Anspruch 5 beanspruchten β-Laktams, das die Verarbeitung einer wie im Anspruch 1 oder 2 beanspruchten Verbindung mit einer Säure umfasst.

**Revendications**

1. β-Lactame ayant la formule générale suivante:

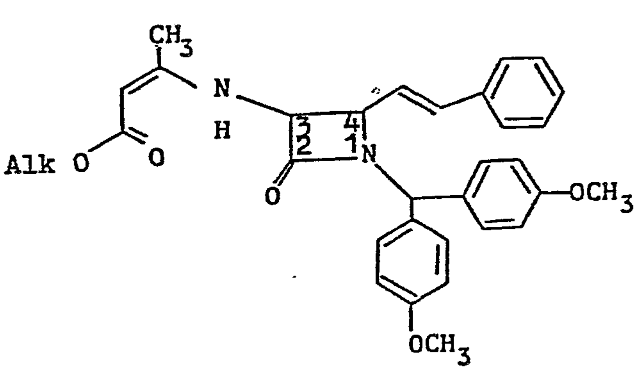

(D)

dans laquelle Alk représente un groupe alkyle ayant de 1 à 4 atomes de carbone.

2. Composé selon la revendication 1, dans laquelle Alk est un groupe méthyle ou un groupe éthyle.

3. Procédé pour la préparation d'un composé tel que revendiqué dans la revendication 1 ou 2, dans lequel un dérivé anhydride d'acide actif d'un composé acide carboxylique ayant la formule générale suivante:

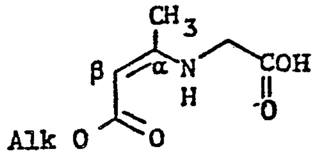

(A)

dans laquelle Alk est comme défini dans la revendication 1 ou 2, est mis en réaction avec un composé azométhine ayant la formule suivante:

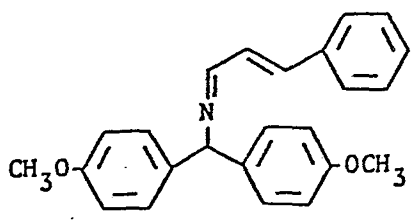

(B)

en présence d'une base.

4. Procédé selon la revendication 3, dans laquelle la base est la triéthylamine, la pyridine ou la lutidine.

5. β-Lactame ayant la formule générale suivante:

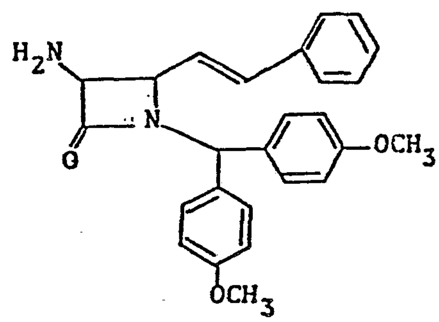

(E)

ou un sel d'addition d'acide de celui-ci.

6. Procédé pour la préparation d'un β-lactame tel que revendiqué dans la revendication 5, qui comprend le traitement d'un composé selon la revendication 1 ou 2 avec un acide.